# EUROPEAN PATENT SPECIFICATION

(11) **EP 0 260 466 B1**
(45) Date of publication and mention of the grant of the patent: **19.07.1995**
(21) Application number: 87112027.5
(22) Date of filing: 19.08.1987
(51) Int. Cl.: A61B 10/00

(54) **Microbiological specimen sampling device**
Vorrichtung zur Entnahme einer Probe für mikrobiologische Zwecke
Dispositif de prise d'un échantillon pour la microbiologie

(30) Priority: 19.09.1986 US 909850
(43) Date of publication of application: 23.03.1988
(73) Proprietor: BOSTON SCIENTIFIC CORPORATION, Natick, MA 01760-1537 (US)
(72) Inventor: Manzo, Michael P., Upton Massachusetts 01568 (US)
(74) Representative: Deans, Michael John Percy

(56) References cited:
- EP-A- 0 087 402
- FR-A- 2 353 640
- GB-A- 2 151 929
- US-A- 4 235 244

## Description

The invention relates to microbiological specimen sampling devices of the type capable of use through the working channel of an endoscope to obtain a specimen within the body.

The sampling device typically consists of a specimen brush disposed within a tubular sheath during introduction and removal from the body. After the physician guides the device through the endoscope inside the body, to a position where it is desired to take a specimen, and while continuing to control the endoscope to view the site, the physician operates the handle of the sampling brush, which extends outside the body, to project the brush distally out of the sheath and work it back and forth within the body to collect the specimen.

Abele et al., in U.S. 4,235,244, for the purpose of keeping the sampling brush sterile until use at the sampling site, taught the use of inner and outer sheaths about the brush, the distal end of the outer sheath initially closed by a plug of water soluble material, e.g., wax, that is discharged within the body at the time it is desired to collect the sample. The plug was ejected at the site by movement of the inner sheath, containing the sampling brush, distally relative to the outer sheath. The device described and illustrated in US-A-4 235 244 comprises an outer catheter in the form of a simple tube with a maximum diameter of about 1.8mm within which is located a slightly smaller simple tubular inner catheter. Within the inner catheter is located a wire connecting the sampling brush to a relatively thin elongate manipulating handle 20 the outer diameter of which, on the scale of the catheter would be a fraction of a millimetre. Manipulation of the illustrated device with its three separate members of outer catheter, inner catheter and sampling wire handle would require the use of both hands. Practical examples of the device described and illustrated in the aforesaid US Patent embodied as the Microbiology Specimen Brush are manufactured by the present applicants and require two-handed operation by a physician to eject the wax plug.

GB-A-2 151 929 describes and illustrates a quite different form of instrument for sampling tissue specimens. This instrument comprises an inner shaft which extends eccentrically through a distally closed outer shaft and receives within it an optical system comprising a lateral objective. The inner shaft is distally connected or connectable to a cutting edge which cooperates with a proximal edge of a distal lateral viewing and cutting aperture in the outer shaft, the edge of the aperture acting as a mating cutting edge over the area of the aperture by axial displacement of the inner shaft within the outer shaft. The outer shaft is provided with a handle in the form of a reduced thickness portion between two relatively enlarged portions. The inner shaft is provided with a handle in the form of a cross-bar mounted on the optical system intermediate its axial ends. The inner and outer shafts are separated by means of a compression spring which biases the inner shaft against movement in the axial direction into the outer shaft. The proximal end of the optical system is also provided with an enlarged surface. No description is given in GB-A-2 151 929 as to how the various parts of the device are grasped in use.

The present invention seeks to provide an improvement to the system of US-A-4 235 244 to enable more ready manipulation and in particular to allow for operation by the digits of one hand.

In accordance with the present invention, there is provided a microbiological specimen handling device comprising a catheter for use within the body, the device comprising: an outer catheter assembly comprising an outer elongate catheter having a distal end and a proximal end; an inner catheter assembly comprising an inner elongate catheter received within the outer catheter assembly and having a distal end and a proximal end, the inner catheter assembly being further provided with a sample collector disposed generally within said inner catheter; a removable water soluble plug engaging and sealing a portion of said outer catheter in a position between the distal end of said outer catheter and the distal end of said inner catheter; and manually operable means for causing relative movement axially between said inner catheter and said outer catheter in a manner to extend said sample collector beyond the distal ends of said catheters to obtain a sample; said device being characterized in that the outer catheter assembly further comprises a longitudinally extending sleeve joined to the proximal end of said outer catheter, which sleeve terminates proximally in an enlarged surface adapted to receive fingers of a physician to support and stabilize the outer catheter assembly; and in that the inner catheter assembly comprises a shaft joined to the proximal end of the inner catheter and having at its proximal end an enlarged portion adapted to receive a digit of a physician's hand whereby to enable relative forward motion of the inner catheter within the outer catheter by the physician squeezing the two enlarged portions together by the digits of one hand, thereby to dislodge said plug from said outer catheter by pushing-out motion of said inner catheter; and in that the inner surface of said sleeve and the outer surface of said shaft are respectively formed with stop means serving to limit longitudinal separation of one said enlarged portion from the other to a predetermined maximum distance within the span of one hand.

The features set out in the abovewritten statement of invention can be related to reference numerals employed in the drawings and in the detailed description in relation to preferred embodiments of microbiological specimen sampling device as follows:
There are described below embodiments of microbiological specimen handling device (10) in accordance with the present invention which comprise a catheter (12,14) for use within the body, the device comprising: an outer catheter assembly (12) comprising an outer elongate catheter having a distal end (38) and a proximal end (16); an inner catheter assembly (14) comprising an inner elongate catheter received within the outer catheter assembly and having a distal end (42) and a proximal end (18), the inner catheter assembly being further provided with a sample collector (44) disposed generally within said inner catheter; a removable water soluble plug (40) engaging and sealing a portion of said outer catheter in a position between the distal end (38) of said outer catheter and the distal end (42) of said inner catheter; and manually operable means (20, 28, 46) for causing relative movement axially between said inner catheter and said outer catheter in a manner to extend said sample collector (44) beyond the distal ends of said catheters to obtain a sample; said device being characterized in that the outer catheter assembly (12) further comprises a longitudinally extending sleeve (16) joined to the proximal end of said outer catheter, which sleeve terminates proximally in an enlarged surface (20) adapted to receive fingers of a physician to support and stabilize the outer catheter assembly (12); and in that the inner catheter assembly (14) comprises a shaft (18) joined to the proximal end of the inner catheter and having at its proximal end (32) an enlarged portion adapted to receive a digit of a physician's hand whereby to enable relative forward motion of the inner catheter within the outer catheter by the physician squeezing the two enlarged portions together by the digits of one hand, thereby to dislodge said plug (40) from said outer catheter by pushing-out motion of said inner catheter; and in that the inner surface of said sleeve and the outer surface of said shaft are respectively formed with stop means (26, 24) serving to limit longitudinal separation of one said enlarged portion from the other to a predetermined maximum distance within the span of one hand.

The invention is further described by way of example only with reference to the following description of a preferred embodiment in which:-
Fig. 1 is a side section view of the microbiological sampling device of the invention as introduced into the patient with the wax plug in place;
Fig. 2 is a similar view of the device with the wax plug ejected;
Fig. 3 is a side plan view of the device with the brush extended distally for sampling; and
Fig. 4 is a somewhat diagrammatic view of the sampling device of the invention in use through a flexible boroscope, while Figs. 4a and 4b are similar views of the device during operation by a physician for ejection of the wax plug and for collection of a specimen, respectively.

The microbiological specimen sampling device 10 of the invention includes an outer catheter 12 and, disposed therewithin, an inner catheter 14, both of Teflon® (polytetrafluroethylene) or other conventional catheter material. The outer catheter is of length sufficient to extend from outside the body to the position within the body where it is desired to obtain a sample, e.g., about 100 cm, and is of outer diameter selected to allow the device to be introduced into the body via the working channel of a flexible endoscope, e.g., the typical working channel has a diameter of about 1.8 mm, and the outer catheter OD is about 1.778 mm (0.070 inch). The inner catheter is about the same length, and has an outer diameter, e.g., about 1.3462 mm (0.053 inch), selected to allow the inner catheter to be moved axially within the outer catheter.

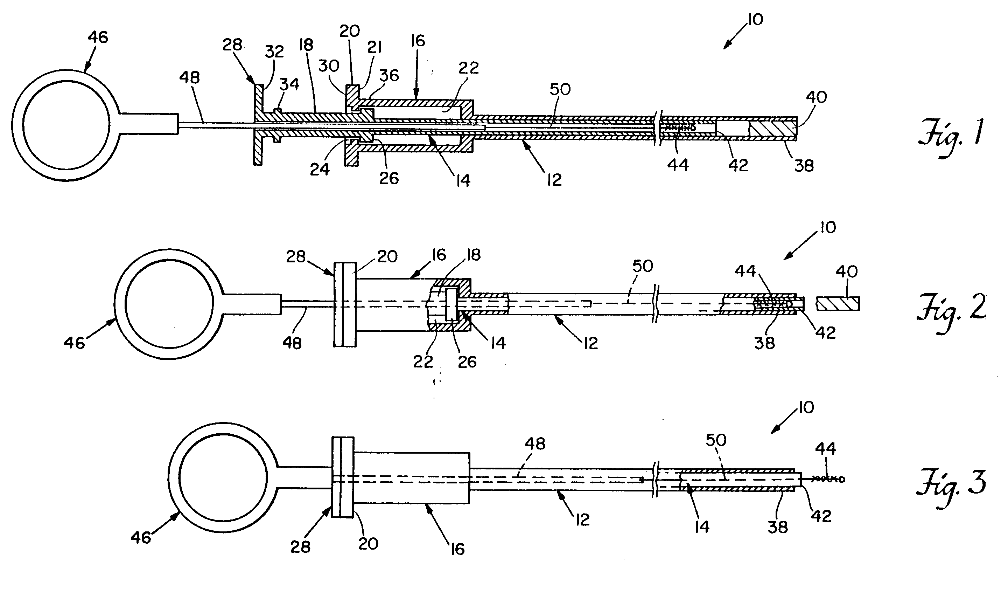

The outer and inner catheters terminate proximally in first and second handle members 16, 18, respectively, both formed of plastic, e.g., modified acrylic, injected molded about the ends of the respective catheters. The first handle member 16 has the form of an elongated sleeve and terminates proximally in a flange 20 defining a radially outwardly extending distal surface 21 about the sleeve. Within member 16 there is defined a center bore 22, restricted in diameter at the proximal end by a radially inwardly extending retaining ring 24. The second handle member 18, connected to the proximal end of inner catheter 14, has the form of an elongated shaft, sized generally to telescope into the center bore of the first handle member, terminating distally in a radial flange 26, and terminating proximally in a radially outwardly extending flange 28. Flange 26 is of diameter sized relative to the inner diameter of the opening in ring 24 to cause the surface of the flange to engage upon the surface of the ring, providing a stop engagement for retaining the shaft within the sleeve when the shaft is drawing proximally relative to the sleeve (Fig. 1). Flange 28 of the second handle member and flange 21 of the first handle member define opposed stop surfaces 30, 32 that engage as the handle shaft telescopes into the handle sleeve to limit axial movement of the inner catheter relative to the outer catheter and position the distal ends of the catheters in the desired relationship (Fig. 2). Defined about the handle shaft, distal of the engaging surface, is an annular rib 34 sized to slide through the center bore of the retaining ring and engage in a snap fit within an annular detent 36 defined by the wall of handle sleeve 16, to hold the handle sleeve and shaft together when the flange surfaces are engaged.

The distal end 38 of the outer catheter 12, as provided to the physician (Fig. 1), is sealed by a plug 40, e.g., about 2 mm in length, of material biologically compatible with the human body, e.g., a water soluble wax or wax-like plug, as described in U.S. 4,235,244 the disclosure of which is incorporated herein by reference. Disposed within the distal end 42 of the inner catheter 14 is a brush 44 for collecting samples of biological material. The sampling brush is connected, through the inner catheter, to a proximal thumbring 46, also of plastic, by a metal hypodermic tubing 48 and by wire 50, the tubing extending proximally from the thumbring, through the handle members and into the proximal end of the inner catheter to provide a stiffener against kinking of the wire during relative movement of the handle sleeve 16, handle shaft 18 and thumbring 46 during obtaining of samples, as we will now describe.

Referring to Fig. 1, the microbiological sampling device 10 of the invention is provided to the physician in the relationship shown, with the distal end 38 of the outer catheter closed by plug 40. The handle shaft member 18 is disposed in its proximal-most position relative to the handle sleeve member 16, with the surfaces of flange 26 and ring 24 engaged. In this position, the distal end 42 of the inner catheter 14 lies proximal of the plug 40, with the brush 44 disposed therewithin, the stiffener tubing 48 extending about an inch from the proximal end of handle shaft member 18. In this condition, the brush and distal portion of the inner catheter are sterile.

The physician now introduces the distal viewing end portion 51 of a flexible endoscope 58 into the body 54 of a patient. Holding the endoscope by handle 60 and viewing through eyepiece 62, the physician, using the steering means 56 provided at the endoscope handpiece, steers the end of the scope to a position, e.g., in the lung 64, where he wishes to obtain a sample. Using his free hand, the physician introduces the distal end 38 of the microbiological sampling device 10, still closed by plug 40, through the endoscope valve 66 and feeds it through the working channel of the scope until the distal end of the device emerges from the distal end of the scope.

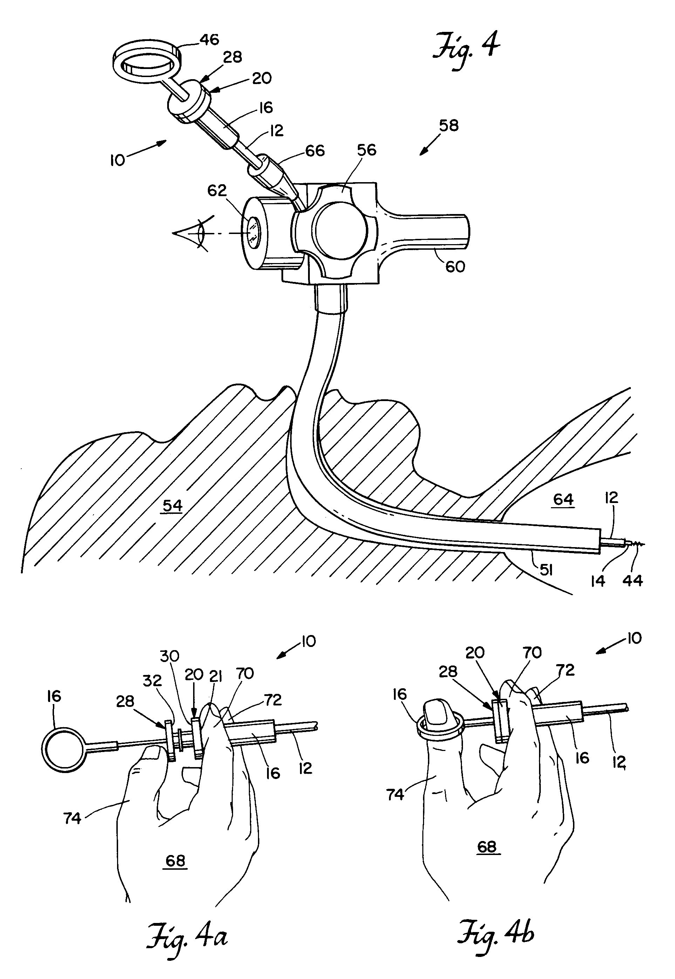

Referring now to Fig. 4a, the physician, using his one free hand 68, grasps the handle sleeve member 16 between two fingers 70, 72, with the fingers resting against distal surface 21 of flange 20. Using the thumb 74 of the same hand, the physician urges the opposed surfaces 30, 32 of the handle sleeve and shaft members toward each other, the shaft telescoping into bore 22, and at the same time advances the distal end of the inner catheter distally relative to the distal end of the outer catheter, the end of the inner catheter engaging against and dislodging plug 40. The physician continues to urge opposed surfaces 30, 32 together until they are engaged, with annular rib 34 about the shaft engaged in snap-fit within annular detent 36 in the wall of the center bore of handle sleeve member 16. In this condition, the distal end of the inner catheter, with the brush 44 still disposed therewithin, extends by a short length beyond the end of the outer catheter, and engagement of rib 34 within detent 36 holds the handle members and the inner and outer catheters in this position, with the sterile brush spaced from the non-sterile outer catheter to avoid contamination (Fig. 2).

Referring now to Fig. 4b, the physician, still using his free hand, moves his thumb 74 from flange 28 to thumbring 46, and, while observing through eyepiece 62, moves the thumbring back and forth to extend the brush 44 distally from within the inner catheter into the body to obtain a sample. During this procedure, the physician continues to grasp the handle sleeve member, into which the handle shaft member is fully telescoped, between his fingers 70, 72 to stabilize the device.

When a sample has been obtained, the sampling brush is withdrawn into the inner catheter, and removed from the body to be evaluated by standard procedures.

## Claims

1. A microbiological specimen handling device comprising a catheter for use within the body, the device comprising: an outer catheter assembly comprising an outer elongate catheter having a distal end and a proximal end; an inner catheter assembly comprising an inner elongate catheter received within the outer catheter assembly and having a distal end and a proximal end, the inner catheter assembly being further provided with a sample collector disposed generally within said inner catheter; a removable water soluble plug engaging and sealing a portion of said outer catheter in a position between the distal end of said outer catheter and the distal end of said inner catheter; and manually operable means for causing relative movement axially between said inner catheter and said outer catheter in a manner to extend said sample collector beyond the distal ends of said catheters to obtain a sample; said device being characterized in that the outer catheter assembly further comprises a longitudinally extending sleeve joined to the proximal end of said outer catheter, which sleeve terminates proximally in an enlarged surface adapted to receive fingers of a physician to support and stabilize the outer catheter assembly; and in that the inner catheter assembly comprises a shaft joined to the proximal end of the inner catheter and having at its proximal end an enlarged portion adapted to receive a digit of a physician's hand whereby to enable relative forward motion of the inner catheter within the outer catheter by the physician squeezing the two enlarged portions together by the digits of one hand, thereby to dislodge said plug from said outer catheter by pushing-out motion of said inner catheter; and in that the inner surface of said sleeve and the outer surface of said shaft are respectively formed with stop means serving to limit longitudinal separation of one said enlarged portion from the other to a predetermined maximum distance within the span of one hand.

2. A device according to Claim 1, further characterized in comprising means for fixing the relative positions of the catheters during obtaining the sample.

3. A device according to Claim 2, further characterized in that said means comprises a rib on the outer surface of said shaft adapted to form a snap fit within a detent formed on the inner proximal surface of the sleeve when the two enlarged surfaces are in engagement.

## Patentansprüche

1. Probenahmegerät für mikrobiologische Proben, umfassend einen Katheter zur Verwendung innerhalb des Körpers, wobei das Gerät umfaßt: einen äußeren Katheteraufsatz, der einen äußeren Verlängerungskatheter mit einem distalen Ende und einem proximalem Ende umfaßt; einen inneren Katheteraufsatz, der einen inneren Verlängerungskatheter umfaßt, der im Inneren des äußeren Katheters aufgenommen wird, und ein distales Ende und ein proximales Ende besitzt, wobei der innere Katheteraufsatz ferner mit einem Probenahmegerät versehen ist, das im allgemeinen innerhalb des inneren Katheters angebracht ist; einen auswechselbaren, wasserlöslichen Verschluß, der in einen Bereich des äußeren Katheters eingreift und ihn abdichtet in einer Position zwischen dem distalen Ende des äußeren Katheters und dem distalen Ende des inneren Katheters; und manuell bedienbare Mittel, die axial eine Relativbewegung zwischen innerem Katheter und äußerem Katheter verursachen, derart, daß sich das Probenahmegerät über die distalen Enden der Katheter hinaus erstreckt, um eine Probe zu erhalten; wobei dieses Gerät dadurch gekennzeichnet ist, daß der äußere Katheteraufsatz ferner ein sich in länglicher Richtung erstreckendes kurzes Rohr umfaßt, das am proximalen Ende des äußeren Katheters befestigt ist, dessen kurzes Rohr proximal in einer erweiterten Oberfläche endet, die so angepaßt ist, daß sie die Finger eines Arztes aufnehmen kann, um den äußeren Katheter zu stützen und zu stabilisieren; und dadurch, daß der innere Katheteraufsatz einen Schaft umfaßt, der am proximalen Ende des inneren Katheters angebracht ist, und an dessen proximalem Ende sich ein erweiterter Bereich befindet, der so angepaßt ist, daß er einen Finger einer Hand eines Arztes aufnehmen kann, wobei er eine relative Vorwärtsbewegung des inneren Katheters innerhalb des äußeren Katheters durch das Zusammendrücken der zwei erweiterten Bereiche durch die Finger einer Hand ermöglicht, wodurch der Verschluß durch die Schubbewegung des inneren Katheters vom äußeren Katheter verdrängt wird; und dadurch, daß die innere Oberfläche des kurzen Rohres und die äußere Oberfläche des Schafts jeweils mit Sperrmitteln versehen sind, die dazu dienen, die longitudinale Trennung eines der erweiterten Bereiche vom anderen auf eine vorherfestgelegte, maximale Entfernung, die innerhalb Spanne einer Hand liegt, zu begrenzen.

2. Gerät nach Anspruch 1, ferner dadurch gekennzeichnet, daß es ein Mittel zur Festsetzung der relativen Postitionen der Katheter während der Pobenahme umfaßt.

3. Gerät nach Anspruch 2, ferner dadurch gekennzeichnet, daß das Mittel eine Rippe auf der äußeren Oberfläche des Schafts umfaßt, die so angepaßt ist, daß sie innerhalb einer Feststellvorrichtung, die auf der inneren, proximalen Oberfläche des kurzen Rohres vorhanden ist, eine Schnappsitz bildet, wenn die zwei erweiterten Oberflachen sich in Eingriff befinden.

## Revendications

1. Dispositif de manipulation de spécimen microbiologique comprenant un cathéter pour une utilisation dans le corps, dispositif comprenant : un ensemble de cathéter externe comprenant un cathéter allongé externe possédant une extrémité distale et une extrémité proximale, un ensemble de cathéter interne comprenant un cathéter allongé interne logé dans l'ensemble de cathéter externe et possédant une extrémité distale et une extrémité proximale, l'ensemble de cathéter interne étant muni, de plus, d'un collecteur d'échantillon placé, de façon globale, dans ledit cathéter interne, un manchon amovible soluble dans l'eau coopérant et obturant une partie dudit cathéter externe dans une position entre l'extrémité distale dudit cathéter externe et l'extrémité distale dudit cathéter interne, et un moyen manuel pour provoquer un déplacement axial relatif entre ledit cathéter interne et ledit cathéter externe de façon à étendre ledit collecteur d'échantillon au-delà des extrémités distales desdits cathéters afin d'obtenir un échantillon;
ledit dispositif étant caractérisé en ce que l'ensemble de cathéter externe comprend, de plus, un manchon s'étendant de façon longitudinale, joint à l'extrémité proximale dudit cathéter externe, manchon se terminant, de façon proximale, en une surface élargie prévue pour recevoir les doigts d'un praticien afin de supporter et de stabiliser l'ensemble de cathéter externe et en ce que l'ensemble de cathéter interne comprend un axe joint à l'extrémité proximale du cathéter interne et possédant, sur son extrémité proximale une partie agrandie prévue pour recevoir un doigt de la main d'un praticien, permettant ainsi un déplacement relatif vers l'avant du cathéter interne dans le cathéter externe, le praticien saisissant les deux parties agrandies à l'aide des doigts d'une main, afin de déloger ainsi ledit bouchon dudit cathéter externe par un déplacement d'extraction dudit cathéter interne et en ce que la surface interne dudit manchon et la surface externe dudit axe sont respectivement formées d'un moyen de butée servant à limiter la séparation longitudinale d'une dite partie agrandie de l'autre d'une distance maximum prédéterminée de la largeur de la main.

2. Dispositif selon la revendication 1, caractérisé, de plus, en ce qu'il comprend un moyen pour fixer les positions relatives des cathéters lors de l'obtention de l'échantillon.

3. Dispositif selon la revendication 2, caractérisé, de plus, en ce que ledit moyen comprend une nervure sur la surface externe dudit axe prévue pour former un encliquetage avec un cliquet formé sur la surface proximale interne du manchon lorsque les deux surfaces agrandies coopèrent.
